# EUROPÄISCHE PATENTANMELDUNG

(11) **EP 1 225 216 A1**
(43) Veröffentlichungstag der Anmeldung: **24.07.2002**
(21) Anmeldenummer: 01100458.7
(22) Anmeldetag: 08.01.2001
(51) Int. Cl.: C12M 1/34

(54) **Vorrichtung zur Untersuchung von Ionenkanälen in Membranen**

(71) Anmelder: Fertig, Niels, 80798 München (DE); Behrends, Jan, 80799 München (DE); Blick, Robert, 81371 München (DE)
(72) Erfinder: Fertig, Niels, 80798 München (DE); Behrends, Jan, 80799 München (DE); Blick, Robert, 81371 München (DE)
(74) Vertreter: Grünecker, Kinkeldey, Stockmair & Schwanhäusser Anwaltssozietät

(57) **Zusammenfassung**

Die Erfindung betrifft Vorrichtungen zur Untersuchung von lonenkanälen in Membranen. Die Erfindung zeichnet sich aus durch einen Biochip mit einem Substrat, in welchem in Form einer M×N Matrix Öffnungen zur Aufnahme einer wenigstens einen lonenkanal (I) umfassenden Zellmembran oder einer künstlichen Lipidmembran (Me) vorgesehen sind, wobei M ≥1 und N≥1,

## Beschreibung

### Gebiet der Erfindung

Die Erfindung betrifft Vorrichtungen und Verfahren zur Untersuchung von lonenkanälen in Membranen, insbesondere Vorrichtungen und Verfahren zur Durchführung der sogenannten Patch-Clamp-Technik mit Hilfe eines Biochips, insbesondere für die Anwendung in Hochdurchsatzverfahren.

### Stand der Technik

lonenkanäle sind Membranproteine, die als schaltbare Poren für Stromfluss dienen. Insbesondere sind lonenkanäle als kleinste erregbare biologische Strukturen die fundamentalen Schaltelemente des Nervensystems. Die Ausstattung einer Nervenzelle mit lonenkanälen verschiedenen Typs bestimmt daher wesentlich ihre Rolle bei der Informationsverarbeitung im Gehirn. Ähnliches gilt im übrigen auch für nicht neuronale erregbare Zellen, beispielsweise für die des Herzmuskels und seiner Erregungsleitungssysteme. Schaltvorgänge in lonenkanälen werden unter anderem deshalb untersucht, um Aufschlüsse über etwaige Fehlfunktionen und deren Behebung durch Medikamente und dergleichen zu gewinnen.

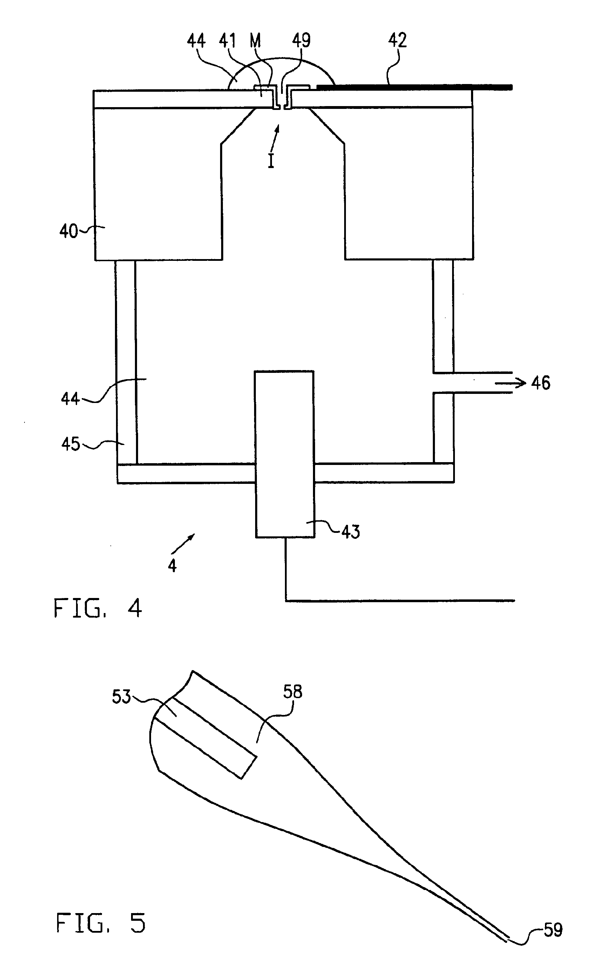

Zur Untersuchung von lonenkanälen in Zellmembranen im Hinblick auf deren Schaltvorgänge, d.h. deren Öffnungs- und Schließmechanismen, wird im Stand der Technik das Patch-Clamp-Verfahren eingesetzt. Hierzu werden sogenannte Patch-Clamp-Pipetten aus Glas verwendet. Eine derartige Pipette ist in Figur 5 dargestellt. Diese Pipette umfasst eine Öffnung 59, die ungefähr einen Durchmesser von 1 µm aufweist. Weiterhin umfasst die Pipette einen Pipettenschaft 58, in dem eine Elektrode 53 vorgesehen ist.

Zur Analyse eines lonenkanals wird ein Membranfleck mittels einer derartigen mit Elektrolyt gefüllten Pipette angesaugt, so dass sich zwischen Membran und Glas ein enger Kontakt bildet. Auf diese Weise wird ein sehr hoher Abdichtwiderstand in einer Größenordnung > 10GΩ erhalten. Hierüber können sehr kleine lonenströme durch die Membran bis hin zu einigen 100 fA gemessen werden.

Nachteil der bekannten Vorrichtung ist jedoch, dass sie nicht geeignet ist, um gleichzeitig eine Vielzahl von Substanzen bzw. die Wirkung einer Substanz auf eine Vielzahl verschiedener (z.B. gentechnisch veränderter) lonenkanäle zu untersuchen. Die bekannte Vorrichtung ist demnach nicht für Hochdurchsatzuntersuchungen geeignet. Dadurch ist diese Vorrichtung nur sehr eingeschränkt zum Substanz-Screening in der pharmazeutischen Industrie einsetzbar.

Weiterer Nachteil der bekannten Vorrichtung ist, dass die Zeitskala, auf der die Öffnungsund Schließmechanismen in lonenkanälen ablaufen, mit dieser Vorrichtung aus Glaspipette, Elektrode und Verstärker nur sehr eingeschränkt zugänglich ist. So besteht für Patch-Clamp-Verfahren mit dieser Vorrichtung eine Beschränkung der Bandbreite auf unter 100 kHz. Zur Untersuchung der Öffnungs- und Schließmechanismen in lonenkanälen wären hingegen Zeitskalen, die einer Bandbreite von > 1 MHz entsprechen, wünschenswert.

Der Erfindung liegt demnach die Aufgabe zugrunde, eine Vorrichtung zur Untersuchung von lonenkanälen in Zellmembranen zu schaffen, welche für Hochdurchsatzverfahren, beispielsweise zum Einsatz in der pharmazeutischen Industrie, geeignet ist und/oder die ein verbessertes Signal-zu-Rauschverhältnis und eine verbesserte Zeitauflösung zeigt.

### Beschreibung der Erfindung

Diese Aufgabe wird durch einen Biochip zur Untersuchung von lonenkanälen mit einem Substrat gelöst, in dem Öffnungen in Form eines M×N-Arrays zur Aufnahme einer wenigstens einen lonenkanal umfassenden Zellmembran oder zur Aufnahme einer wenigstens einen lonenkanal umfassenden künstlichen Lipidmembran vorgesehen sind, wobei M ≥ 1 und N ≥ 1.

Durch Verwendung eines derartigen Biochips kann auf die Pipette, deren relativ langer Schaft zu einer hohen Streukapazität führt, verzichtet werden. Vielmehr können von vornherein die kritischen geometrischen Parameter optimiert werden, wodurch das Signal-zu-Rauschverhältnis gegenüber dem Stand der Technik stark verbessert wird und demnach die Zeitauflösung erhöht wird. Dies gilt sowohl für Biochips mit einer einzigen Öffnung, d.h. für M = N = 1, als auch für Biochips mit einer Mehrzahl von Öffnungen, d.h. M > 1 und/oder N > 1.

Durch die Mehrzahl der Öffnungen zur Aufnahme von Membranen, die lonenkanäle enthalten, kann darüber hinaus im Fall M > 1 und/oder N > 1 die Patch-Clamp-Technik parallelisiert werden, wodurch sich M mal N Messungen simultan mit einem Chip vornehmen lassen.

Besonders vorteilhaft ist hierbei die Anpassung der Form dieses M×N-Arrays an die Geometrie der in den pharmazeutischen Industrie standardmäßig verwendeten 96-Küvettenplatten. Diese Küvettenplatten lassen sich in Pipetierautomaten einsetzen, mit denen Substanzen auf den hier beschriebenen Biochip vorteilhaft appliziert werden können.

Darüber hinaus ermöglicht der erfindungsgemäße Biochip, bedingt durch seine Geometrie, dass auf ihm aufgebrachte Membranen gegenüber der bekannten Vorrichtung wesentlich leichter zugänglich sind. So lassen sich die Membranen wesentlich besser beobachten, chemisch und/oder mechanisch und/oder elektrisch manipulieren.

Gemäß einer vorteilhaften Weiterbildung des zuvor beschriebenen Biochips weist die Oberfläche im Bereich einer jeden Öffnung aufnahmeseitig eine Einrichtung zur Verbesserung des Kontaktes mit der Zellmembran auf, durch die eine verbesserte Haftung der Membran an dem Biochip gewährleistet werden kann. Hierdurch lässt sich auch der elektrische Abdichtwiderstand erhöhen.

Entsprechend einer vorteilhaften Weiterbildung kann die Einrichtung zur Verbesserung des Kontaktes in Form einer Strukturierung der Oberfläche ausgebildet sein.

Hierzu kann die Strukturierung in Form eines oder einer Mehrzahl von Ringen, der oder die um jede Öffnung angeordnet sind, oder in Form eines oder einer Mehrzahl von Quadraten oder Rechtecken, das oder die um jede Öffnung angeordnet sind, vorgesehen sein.

Insbesondere kann die Strukturierung hierbei in Form einer um die Öffnung in geringem Abstand konzentrisch angeordneten Vertiefung in der Oberfläche des Biochips vorgesehen sein, deren Durchmesser ein Mehrfaches des Durchmessers der Öffnung beträgt, so dass der Rand der Öffnung aus dem sie umgebenen Niveau des Biochips nach oben herausragt. Auf diese Weise wird eine Zellemembran durch den Rand der Öffnung eingedellt, was zur Erhöhung des Kontakts zwischen Biochip und Membran führt.

Jede Öffnung kann Längen- und Breitenabmessungen aufweisen, die in einem Bereich von 10 µm bis 10 nm liegen. Hierdurch lässt sich die Anzahl der beobachteten lonenkanäle einstellen. Des weiteren wird durch eine kleinere Öffnung auch die Membranfläche und damit die Kapazität verringert, was zu einer weiter verbesserten Messauflösung führt.

Der erfindungsgemäße Biochip eignet sich auch hervorragend zur Ausbildung künstlicher Lipidmembranen (künstliche Lipiddoppelschicht) auf der Öffnung, und zwar in Analogie zum bekannten Black-Lipid- oder Lipid-Bilayer-Verfahren. Hierdurch wird die Untersuchung von lonenkanälen durch Fusion lonenkanäle enthaltender Vesikel mit der künstlichen Lipiddoppelschicht ermöglicht.

Aufgrund der im Vergleich zum bekannten Bilayer-Verfahren geringen Größe der Apertur (bei bekannten Vorrichtungen ist die Größe der Apertur regelmäßig > 100 µm) und der daraus resultierenden geringen Kapazität lässt sich das Signal-Rauschverhältnis verbessern.

Entsprechend einer bevorzugten Ausführung der zuvor beschriebenen Biochips kann jede Öffnung im wesentlichen kreisförmig sein. Derartige Kreisformen lassen sich auf einfache Weise in den Biochip implementieren. Falls eine einfache Implementierung nicht erforderlich ist, können auch andere Formen für die Öffnungsquerschnitte gewählt werden.

Entsprechend einer vorteilhaften Weiterbildung aller zuvor beschriebener Biochips kann das Substrat einen Basisabschnitt mit einer ersten Dicke und einen bzw. eine Mehrzahl von im Basisabschnitt ausgebildeten Fensterabschnitt bzw. Fensterabschnitten mit einer zweiten Dicke aufweisen, in dem bzw. in denen jeweils eine Öffnung vorgesehen ist. Insbesondere können hierbei die Dicke des Basisabschnitts in einem Bereich von 1 mm bis 100µm und die Dicke des Fensterabschnitts in einem Bereich von 1µm bis 50 µm liegen. Durch diese Weiterbildung bleibt die mechanische Stabilität des Substrats gewährleistet, während die Länge der Apertur (senkrecht zum Öffnungsquerschnitt) und damit auch der elektrische Zugangswiderstand möglichst gering bleiben.

Entsprechend einer vorteilhaften Weiterbildung aller zuvor beschriebener Biochips kann das Substrat ein Halbleitermaterial, wie GaAs, Si, oder AlGaAs oder Glas oder Quarz oder Polymere, wie Polydimethylsiloxan (PDMS) umfassen. Durch diese Materialen lassen sich eine Vielzahl von Vorteilen, insbesondere eine einfache Herstellung durch eine für das jeweilige Material ausgereifte Prozesstechnik, erzielen.

Bei Verwendung eines Substrats aus Halbleitermaterial, insbesondere Si, GaAs oder AlGaAs, kann eine passivierende und isolierende Schicht, die einseitig oder beidseitig auf das Substrat aufgebracht wird, vorgesehen werden. Diese Isolationsschicht kann insbesondere aus SiO₂,Si₃N₄, Glas oder Polymeren, sowie aus Mehrschichtsystemen bestehen, in denen diese Materialien miteinander und/oder mit den oben genannten Halbleitern und/oder mit Metallen kombiniert werden, und Dicken von 50 nm bis zu mehreren µm aufweisen. Mit diesen Materialien kann ein Abdichtwiderstand von einigen GΩ, wie er zur Messung von Strömen im pA-Bereich erforderlich ist, realisiert werden.

Die Isolationsschicht kann bei der Herstellung dieser Ausführungsform auch die Funktion einer Ätzstoppschicht erfüllen, und bei anisotropem Ätzen des Halbleiters zur Ausbildung eines Fensterabschnitts führen, in dem nur noch die Isolationsschicht vorhanden ist. Die Apertur kann dann lithographisch definiert und in die freitragende Isolationsschicht durch Trockenätzverfahren eingebracht werden.

Als weitere vorteilhafte Alternative lassen sich Polymere, wie Polydimethylsiloxan (PDMS) als Substratmaterial einsetzen. Bei der Herstellung des zuvor beschriebenen Biochips aus PDMS wird ein 3D-Negativtemplat (Gussform) verwendet, das die invertierte Struktur des gewünschten Biochips hat. Das PDMS ist zunächst zähflüssig und wird, nach Vermischen mit Härter, in die Gussform gegossen und mit oder ohne Erhitzen (etwa 60 bis 100 Grad Celsius) ausgehärtet. Der flexible Biochip kann daraufhin aus der Gussform ausgelöst werden, wobei eine vorherige Beschichtung der Gussform mit Silanen das Ablösen erleichtern kann. Für die Herstellung dieser Ausführungsform ist eine chemische Modifikation der Oberflächen (insbesondere Oxidation im Plasmaverascher aber auch andere geeignete Verfahren) vorteilhaft.

Darüber hinaus können sämtliche Oberflächen des Biochips zusätzliche isolierende und passivierende Schichten aus den bereits erwähnten Materialien, sowie chemische Modifikationen (Silanisierung, Oxidation) aufweisen.

Die zuvor beschriebenen Biochips lassen sich auf einfache Weise herstellen. Grundsätzlich sind allen Verfahren folgende Schritte gemeinsam: Vorsehen eines Substrats, Ausbilden eines oder mehrerer Fensterabschnitte in dem Substrat, und Ausbilden je einer Öffnung pro Fensterabschnitt.

Im Falle eines Biochips auf Basis eines Halbleitersubstrat mit Isolationsschicht bietet sich für die Herstellung des Fensterabschnitts folgendes Verfahren an; eine dem nasschemischen Ätzverfahren (insbesondere KOH) gegenüber resistente, ober- und unterseitig vorhandene Isolationsschicht wird unterseitig durch einen Trockenätzschritt in einem lithographisch definierten Bereich entfernt, wodurch in diesem Bereich das Halbleitersubstrat direkt exponiert ist. Der folgende nasschemische Ätzschritt (insbesondere KOH) bewirkt dann durch anisotropes Ätzen die Ausbildung eines Ätzgrabens, der die Form einer umgekehrten Pyramide hat. Dieser Ätzgraben kann bei ausreichender Größe der primär exponierten Substratfläche bis auf die gegenüberliegende Seite reichen, bleibt jedoch in jedem Falle durch die gegenüberliegende, dem nasschemischen Ätzmittel gegenüber resistente Isolationsschicht, die also als Ätzstoppschicht wirkt, einseitig verschlossen. Hierdurch kann auf sehr einfache Weise eine präzise Ausbildung eines rechteckigen Fensterbereichs erhalten werden, dessen Fläche von der Fläche des unterseitig im ersten Schritt exponierten Substrats abhängt. Als Ätzstoppschicht bzw. Isolationsschicht haben sich hierbei insbesondere eine Si₃Nₓ-Schicht, vorzugsweise eine Si₃N₄-Schicht, eine SiO₂-Schicht oder Si₃Nₓ/ SiO₂-Mehrschichtssysteme als günstig erwiesen.

Schließlich kann durch optische Lithographie und einen Trockenätzschritt im Fensterabschnitt die Öffnung selbst gebildet werden. Dieses Verfahren eignet sich für vergleichsweise große Öffnungen (größer gleich 1 µm). Sollen kleinere Öffnungen, d.h. bis herab zu 10 nm vorgesehen werden, lässt sich die Öffnung beispielsweise durch Elektronenstrahllithographie und einen Trockenätzschritt bilden.

Bei Ausführung des Biochips auf Glassubstrat oder Quarzsubstrat kommt ein isotropes HF-Ätzverfahren zur Definition des Fensterabschnitts durch lokale Ausdünnung des Glassubstrats zum Einsatz. Die eigentliche Öffnung kann durch Lithographie in Kombination mit einem Trockenätzschritt in das Fenster eingebracht werden. Im Fall dieser Substratmaterialien kann auch das Aufätzen einer latenten Spur eines einzelnen hochenergetischen lons, das den ausgedünnten Fensterbereich durchlaufen hat, zur Herstellung der Apertur genutzt werden.

Bei einer Ausführung des Biochips aus PDMS entfällt der Ätzschritt, da hier mit einem Abdruckverfahren gearbeitet wird; d.h. sowohl Fensterabschnitt als auch Öffnungen werden von einem 3D-Negativtemplat übertragen. Die beschriebenen Ätz- und Lithographieverfahren kommen allerdings bei der Herstellung des Negativtemplats zum Einsatz.

Gemäß einer bevorzugten Weiterbildung aller zuvor beschriebenen Biochips können integrierte Elektroden auf einer oder auf beiden Seiten des Substrats vorgesehen sein. Insbesondere lassen sich Elektroden, z.B. aus Gold, Silber oder anderen geeigneten Metallen, direkt auf den Chip aufdampfen. Dies vereinfacht den Versuchsaufbau, da die Elektroden bereits fest auf dem Biochip integriert sind und demnach ein Anbringen und Justieren der Elektroden entfällt. Außerdem können durch eine derartige Anordnung, insbesondere wenn die Elektroden bis auf wenige µm an die Membran herangeführt werden, die parasitären Kapazitäten und Widerstände noch stärker verringert werden, was zu einer weiteren Verbesserung des Signal-zu-Rauschverhältnisses führt.

Ob ein Biochip mit integrierten Elektroden auf einer oder auf beiden Seiten des Substrats zum Einsatz kommt, kann in Abhängigkeit von dem durchzuführenden Versuch bestimmt werden. Als Elektroden eignen sich insbesondere Ag/AgCl-Elektroden. Diese Elektroden haben den Vorteil, dass eine Elektrodenpolarisierung, die zur Verfälschung der Messergebnisse führen würde, vermieden wird.

Außerdem können zusätzliche Elektroden integriert werden, dass über die hochfrequente elektromagnetische Wechselfelder anlegbar sind. Insbesondere durch Anlegen eines hochfrequenten Wechselfelds im Bereich von MHz bis GHz lässt sich die Dynamik der lonenkanäle beeinflussen bzw. analysieren. Zum Anlegen derartiger hochfrequenter Felder ist die Verwendung von Antennenstrukturen (z.B. die aus der Hochfrequenztechnik bekannte Bow-Ti-Antenne) besonders geeignet. Hierdurch kann eine effektive Kopplung des elektromagnetischen Feldes an den lonenkanal erzielt werden.

Die Elektroden können hierbei eine Breite von 40 nm aufweisen und die Elektroden können bis auf wenige nm an die Öffnung herangeführt sein, um die Einkopplung der Leistung der Wechselfelder zu optimieren.

Ebenso wie Elektroden können gemäß einer bevorzugten Weiterbildung der zuvor beschriebenen Biochips auch elektrisch und/oder optisch aktive und/oder passive Bauelemente auf dem Substrat integriert sein. Hierdurch ergibt sich ein weitere strukturelle Vereinfachung des Versuchsaufbaus. Insbesondere können so auch die Signalwege kurz gehalten werden, was sich wiederum auf das Signal-zu-Rauschverhältnis günstig auswirkt. So können die Biochips beispielsweise integrierte Feldeffekttransistoreinrichtungen zur Vorverstärkung von Messsignalen aufweisen.

Die Elektroden, die elektrisch und/oder optisch aktive und/oder passive Bauelemente können vorteilhafter weise auf dem Substrat, gegebenenfalls auf der Ätzstoppschicht bzw. Isolationsschicht, integriert werden.

Entsprechend weiterer bevorzugter Ausführungsformen können in allen zuvor beschriebenen Biochips optische Nahfeldeinrichtungen zur Beobachtung des oder der lonenkanäle vorgesehen sein. Die Möglichkeit Nahfeldeinrichtungen einzusetzen ergibt sich aus der geometriebedingt leichten Zugänglichkeit einer Membran auf dem Biochip. Insbesondere können deshalb alle Rastersondenverfahren, wie Rasterkraftmikroskopie (AFM), Optische Rasternahfeldmikroskopie (SNOM) und Rastertunnelmikroskopie (STM) problemlos zur Beobachtung der Membranen verwendet werden.

Darüber hinaus können aufgrund der geometriebedingt leichten Zugänglichkeit auch andere bildgebende Verfahren, wie beispielsweise Rasterelektronenmikroskopie (REM), konfokale Fluoreszenzmikroskopie (auch in Kombination mit SNOM), Fluoreszenzspektroskopie, optische Mikroskopie oder Einzelphotonendetektion eingesetzt werden. Insbesondere die Ausführung des Biochips in Glas oder Polydimethylsiloxan (PDMS) ist für Fluoreszenzuntersuchungen geeignet, da hier das Substrat einen geringen Fluoreszenzhintergrund aufweist.

Vorteilhafterweise können in den zuvor beschriebenen Biochips Mikrofluidkanäle zur Perfusion vorgesehen sein.

Alle zuvor beschriebenen vorteilhaften Weiterbildungen lassen sich sowohl bei Biochips mit einer Öffnung (M = N = 1) als auch bei Biochips mit einer Mehrzahl von Öffnungen (M > 1 und/oder N > 1) einsetzen.

Alle zuvor beschriebenen Biochips lassen sich, abgesehen von herkömmlichen Untersuchungen von lonenkanälen in Membranen auf vielfältigste Weise einsetzen.

In die Öffnung oder die Öffnungen des Biochips können analog der aus der Patch-Clamp-Technik bekannten sog. exzidierten Membranpatches Teilbereiche der Zellmembran von Zellen (z.B. isolierte Zellen aus Geweben oder Primärkulturen, sowie Zelllinien, die bestimmte lonenkanäle exprimieren) inkorporiert werden. Es kann über die Apertur analog der aus der Patch-Clamp-Technik bekannten sogenannten Ganzzellspannungsklemme auch Kontakt zum Innern einer ganzen Zelle hergestellt werden.

Weiterhin können alle beschriebenen Biochips auf der der Aufnahmeseite gegenüberliegenden Unterseite mit Vorrichtungen versehen werden, die das einfache Anlegen eines Unter- oder Überdruckes gegenüber der Oberseite (d.h. eines Druckgradienten über die Aperturen) gestatten. Diese können beispielsweise als unter jeweils jeder Öffnung bzw. jedem Fensterbereich befindliche, flüssigkeitsgefüllte Hohlkammern in einem flexiblen Polymersubstrat (z.B. PDMS) ausgebildet sein, die jeweils mit der Apertur und durch sie mit der Oberseite des Biochips verbunden sind, und deren Volumen durch von außen wirkenden, durch eine mechanische Vorrichtung erzeugten Druck verkleinert, bzw. durch Verringern desselben wieder vergrößert werden kann.

Das Anlegen eines Druckgradienten über die Aperturen kann auch über Mikrofluidkanäle und damit verbundene Schlauchsysteme erfolgen.

Die erfindungsgemäßen Biochips, können auch in einer Messsonde eingesetzt werden, mit einem Glasröhrchen, das auf der Seite des Substrats vorgesehen ist, die der Seite, auf der die Membran aufbringbar ist, gegenüberliegt, wobei die dem Substrat abgewandte Öffnung des Glasröhrchens so ausgebildet ist, dass eine Elektrode einführbar ist. Hierdurch kann eine Elektrode in ionischer Lösung zur Untersuchung des oder der lonenkanäle an die Öffnung herangeführt werden.

Alternativ dazu kann statt eines Glasröhrchens eine Haltevorrichtung aus Polycarbonat oder einem anderen Material außer Glas vorgesehen sein, die über einen zentralen Hohlraum oder mehrere Hohlräume verfügt, der mit der Apertur bzw. die mit den Aperturen des Biochips kommunizieren und auf welche der Biochip aufgeklebt oder anders befestigt wird und in das eine Elektrode bzw. mehrere Elektroden in ionischer Lösung einführbar ist bzw. sind

So entsteht eine Einrichtung, in welcher der oben beschriebene Biochip auf einfache Weise integriert werden kann. Insbesondere kann diese Messsonde auch problemlos in bekannten Patch-Clamp-Aufbauten, insbesondere in aufrechte und invertierte optischen Mikroskopen sowie Messplätzen für optische und mechanische Rastersondenverfahren integriert werden.

Vorteilhafterweise kann in einer solchen Messsonde die dem Substrat abgewandte Öffnung des Glasröhrchens oder der Haltevorrichtung so ausgebildet sein, dass eine Elektrodeneinrichtung einschraubbar ist. In dieser Anordnung kann die Elektrodeneinrichtung schnell gewechselt werden und ist darüber hinaus wieder verwendbar. Diese Anordnung eignet sich unter anderem für einen Biochip, der integrierte Elektroden nur auf der Oberseite des Chips aufweist.

Die Messsonde kann zweckmäßigerweise auch zusammen mit der einschraubbaren Elektrode vertrieben werden.

In einer derartigen Anordnung sind zweckmäßigerweise zwischen der Öffnung des Glasröhrchens und der einschraubbaren Elektrode Dichtungsmittel, beispielsweise O-Ringe, vorgesehen, damit der Elektrolyt im Glasröhrchen bzw. in der Haltevorrichtung verbleibt.

Vorteilhafterweise ist das Glasröhrchen oder die Haltevorrichtung mit dem Substrat verklebt oder mit einem Dichtungsring mit dem Substrat verschraubbar. Hierdurch kann eine einfache und dichte Verbindung zwischen Glasröhrchen und Substrat sichergestellt werden. Die Verschraubung gemäß der zweiten Alternative führt zusätzlich zur einfachen Wiederverwertbarkeit des Biochips, da sie eine agressive Reinigung des Biochips ermöglicht.

Die zuvor beschriebenen Messsonden können vorteilhaft so vorgesehen werden, dass sie eine Einrichtung zum Erzeugen von Unterdruck in dem Glasröhrchen bzw. in der Haltevorrichtung aufweisen. Mit dieser Einrichtung kann mit der üblichen Ansaugtechnik ein Membranfleck einer ebenfalls in Lösung befindlichen Zelle definiert werden. Damit können alle zur Durchführung einer Analyse von lonenkanälen erforderlichen Schritte an einer einzigen Vorrichtung durchgeführt werden. Dies führt zu einer verbesserten Handhabbarkeit der Vorrichtung.

Im folgenden werden besondere Ausführungsformen der Erfindung unter Bezugnahme auf die beigefügte Zeichnung erläutert. In der Zeichnung zeigen:

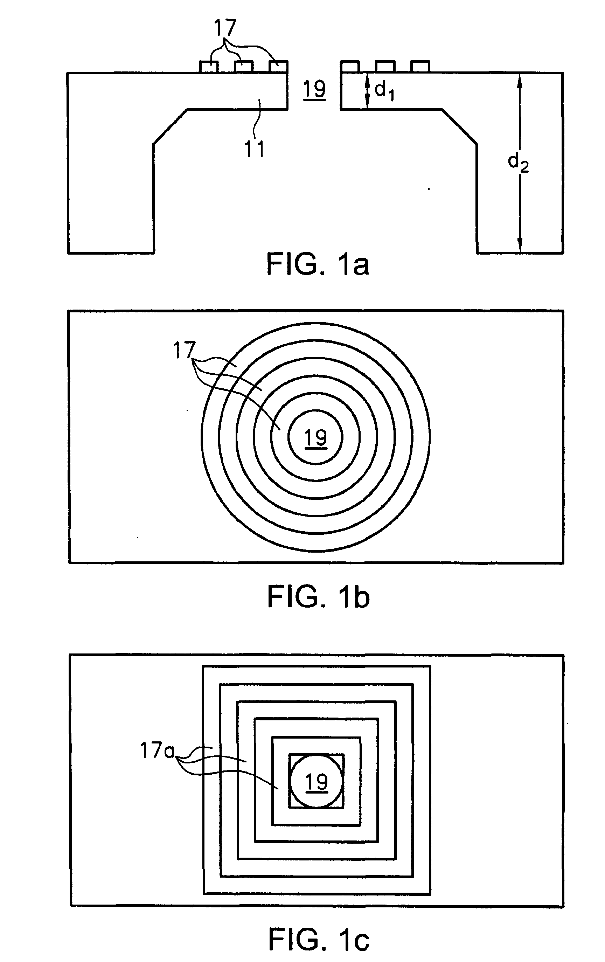

- Fig. 1a: eine Schnittansicht einer ersten Ausführungsform eines Biochips gemäß der vorliegenden Erfindung;
- Fig. 1b: eine Draufsicht auf die erste Ausführungsform eines Biochips gemäß der vorliegenden Erfindung;
- Fig. 1 c: eine Draufsicht auf eine Abwandlung der ersten Ausführungsform eines Biochips gemäß der vorliegenden Erfindung;
- Fig. 2: eine zweite Ausführungsform des Biochips gemäß der vorliegenden Erfindung;
- Fig. 3: eine dritte Ausführungsform des Biochips gemäß der vorliegenden Erfindung;
- Fig. 4: eine Ausführungsform der Messsonde gemäß der vorliegenden Erfindung; und
- Fig. 5: eine Pipette zur Untersuchung von lonenkanälen gemäß dem Stand der Technik.

Figur 1a und 1b zeigen eine erste Ausführungsform 1 eines Biochips gemäß der vorliegenden Erfindung.

Dieser Biochip umfasst ein Substrat, in dem eine Öffnung 19 zur Aufnahme einer wenigstens einen lonenkanal umfassenden Zellmembran vorgesehen ist. Im vorliegenden Fall ist ein Biochip mit M = N = 1 dargestellt.

Das Substrat umfasst einen Basisabschnitt 10 mit einer ersten Dicke d₁ und einen Fensterabschnitt 11 mit einer zweiten Dicke d₂, in dem die Öffnung 19 vorgesehen ist.

Die Dicke des Basisabschnitts 10 liegt in einem Bereich von 1 mm bis 100 µm und die Dicke des Fensterabschnitts liegt in einem Bereich von 1 µm bis 50 nm. Der Fensterabschnitt hat hierbei eine Fläche von einigen 10 µm².

Die Öffnung 19 ist im wesentlichen kreisförmig und hat einen Durchmesser, der in einem Bereich von 10 µm bis 10 nm liegt. Die Größe der Öffnung bestimmt sich danach, wie viele lonenkanäle in einer Zellmembran untersucht werden sollen.

Der Biochip 1 ist aus einem (0001)-Quarz (Z-Schnitt) gebildet, in den zunächst durch einen anisotropen nasschemische Ätzschritt der Fensterabschnitt 11 ausgebildet wird. Als Ätzmittel wird hierbei HF verwendet.

Im letzten Schritt wird, je nach Größe der erwünschten Öffnung, diese Öffnung durch optische Lithographie und einen Trockenätzschritt oder durch Elektronenstrahllithographie und einen Trockenätzschritt gebildet.

Weiterhin ist die Oberfläche des Biochips gemäß Figur 1 im Bereich der Öffnung mit einer Einrichtung zur Verbesserung des Kontaktes zwischen Biochip und Zellmembran versehen. Diese Einrichtung ist vorliegend durch eine Strukturierung der Oberfläche ausgebildet. Hierzu sind ringförmige Erhebungen 15, die um die Öffnung angeordnet sind, vorgesehen.

Durch diese Erhebungen wird eine zu untersuchende Membran mit einem lonenkanal eingedellt, wodurch aufgrund eines Hydraulikeffekts eine bessere Haftung erzielt wird und der elektrische Abdichtwiderstand erhöht wird.

Die Strukturierung in dem Biochip gemäß Figur 1a und 1b ist hierbei lediglich beispielhaft zu verstehen. Insbesondere lassen sich auch andere Formen von Erhöhungen einsetzen, beispielsweise ein oder eine Mehrzahl von Quadraten oder Rechtecken, das oder die um jede Öffnung angeordnet sind. Eine dieser Alternativen ist in Figur 1c dargestellt.

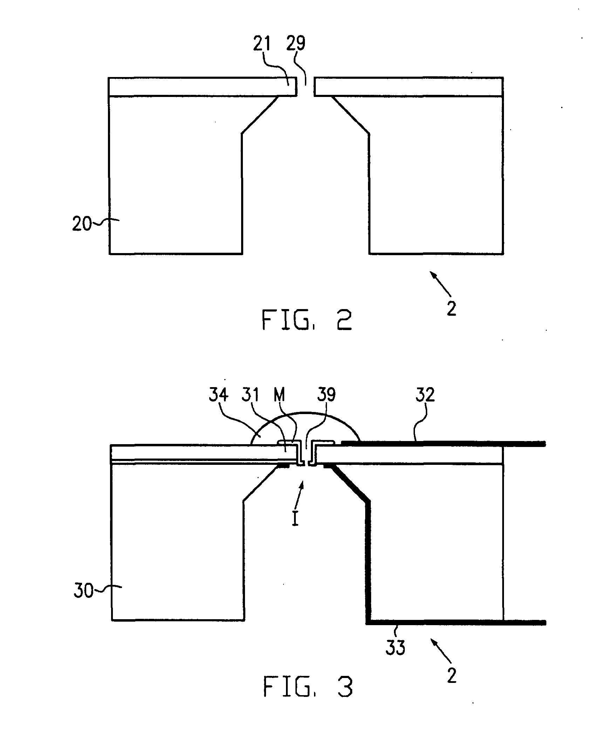

Figur 2 zeigt eine zweite Ausführungsform eines Biochips gemäß der vorliegenden Erfindung.

Dieser Biochip weist ebenfalls ein Substrat 20, 21, in dem eine Öffnung 29 zur Aufnahme einer wenigstens einen lonenkanal umfassenden Zellmembran vorgesehen ist. Auch für den in Figur 2 dargestellten Biochip ist M = N = 1.

Die geometrische Form und die Abmessungen des Biochips 2 entsprechen denen des in Figur 1 gezeigten Biochips 1. Zur Vermeidung von Wiederholungen wird in diesem Zusammenhang lediglich auf die entsprechende Beschreibung der Figur 1 verwiesen. Die Bezugszeichen einander entsprechender Teile unterscheiden sich hierbei nur in ihrer ersten Ziffer.

Das Substrat des Biochips 2 umfasst einen Basisabschnitt 20, der ebenfalls aus Quarz gebildet ist, und eine Ätzstoppschicht, in welcher der Fensterabschnitt 21 ausgebildet ist. Diese Ätzstoppschicht besteht aus Si₃Nₓ, vorzugsweise Si₃N₄.

Gegenüber dem Biochip 1 auf Figur 1 zeichnet sich der Biochip 2 dadurch aus, dass er durch ein vereinfachtes Verfahren herstellbar ist.

So wird auf das Substrat 20 zunächst ein Ätzstoppfilm aufgebracht. Anschließend wird von der gegenüberliegenden Seite durch einen anisotropen nasschemischen HF-Ätzschritt der Fensterabschnitt 11 bis zur Ätzstoppschicht gebildet. Abschließend wird die Öffnung, vorzugsweise durch eines der im Zusammenhang mit der ersten Ausführungsform beschriebenes Verfahren, gebildet.

In Figur 3 ist eine dritte Ausführungsform eines Biochips 3 gemäß der vorliegenden Erfindung dargestellt.

Bezüglich der geometrischen Abmessungen und Aufbaus entspricht der Biochip 3 im wesentlichen dem Aufbau der in den Figuren 1 und 2 beschriebenen Biochips, so dass auch hier zur Vermeidung von Wiederholungen auf die Beschreibung dieser Chips verwiesen wird. Die Bezugszeichen einander entsprechender Teile unterscheiden sich hierbei nur in ihrer ersten Ziffer.

Im Gegensatz zu den dort gezeigten Biochips besteht der Basisabschnitt 30 des Substrat aus einem Halbleitermaterial, beispielsweise (100)-Si.

Auf diesem Halbleitermaterial ist eine isolierende Schicht aufgebracht, in welcher der Fensterabschnitt 31 ausgebildet ist. Die isolierende Schicht 31 dient außerdem im Herstellungsverfahren als Ätzstoppschicht.

Der Herstellungsvorgang ist demnach dem in Figur 2 hergestellten Biochip ähnlich. In der dargestellten Ausführungsform besteht diese Schicht aus Si₃N₄.

Insbesondere wird zuerst auf den Siliziumbasisabschnitt 30 die Isolations- und Ätzstoppschicht mit einem PECVD-Verfahren aufgebracht. Dann wird von der anderen Seite durch einen anisotropen nasschemischen KOH-Ätzschritt der Fensterabschnitt 31 in dem Substrat ausgebildet. Hierbei wird bis zur Ätzstoppschicht durchgeätzt. Anschließend kann, wie in den zuvor beschriebenen Ausführungsformen in Abhängigkeit von der erwünschten Größe der Öffnung diese durch optische Lithographie bzw. Elektronenstrahllithographie und einen Trockenätzschritt ausgebildet werden.

Im letzten Schritt werden schließlich noch Elektroden 32 und 33, die im vorliegenden Fall aus Ag/AgCl bestehen, auf die Oberseite und die Unterseite des Substrats aufgebracht.

In Figur 3 ist darüber hinaus dargestellt, wie eine Membran Me mit einen lonenkanal I in die Öffnung 39 eingebracht worden ist. Zur anschließenden Messung, die unter Bezugnahme auf Figur 4 noch im Detail beschrieben wird, muss eine Elektrolytflüssigkeit 34 über Membran und Elektrode 32, sowie im Ätzgraben, vorgesehen werden.

In den Figuren 1 bis 3 sind jeweils Biochips mit M = N = 1 dargestellt. Selbstverständlich gilt oben Gesagtes auch für Biochips mit Substraten, in denen eine Mehrzahl von Öffnungen vorgesehen sind. Diese Öffnungen können in Form eines M × N-Arrays vorgesehen sein. Hierbei können sie regelmäßig oder reihenweise versetzt angeordnet werden.

Die in den Figuren 1 bis 3 dargestellten Biochips stellen lediglich bevorzugte Ausführungsformen der Erfindung dar, und sind nicht als Beschränkung derselben zu verstehen.

Dem gemäß sind eine Vielzahl anderer, nicht gezeigter Ausführungsformen möglich.

Beispielsweise ist es nicht erforderlich, dass die Öffnung kreisförmig ausgebildet ist. Sie kann je nach Anforderung verschiedene Querschnitte aufweisen.

Außerdem lassen sich verschiedene Materialien zur Ausbildung der Biochips einsetzen. So kann beispielsweise anstelle des Quarzes auch Glas und anstelle des Silizium ein anderes Halbleitermaterial, beispielsweise GaAs, verwendet werden.

Insbesondere im Fall eines Substrats aus Halbleitermaterial, allerdings nicht beschränkt hierauf, können die Oberflächen des Substrats mit einer passivierenden Schicht überzogen werden.

Weiterhin lassen sich auch verschiedenartige Elektroden einsetzen, beispielsweise solche, die zur Erzeugung eines elektromagnetischen Feldes im Bereich des lonenkanals geeignet sind.

Darüber hinaus können elektrisch und/oder optisch aktive und/oder passive Bauelemente auf dem Substrat integriert werden.

Ebenso lassen sich zur Herstellung der Biochips verschiedene aus der Halbleitertechnologie hinreichend bekannte Verfahren in Abhängigkeit von dem jeweils verwendeten Materialien einsetzen.

In Figur 4 ist eine Messsonde gemäß einer Ausführungsform der vorliegenden Erfindung dargestellt.

Diese Messsonde umfasst ein Substrat mit einem Basisabschnitt 40 und einem Fensterabschnitt 41, in dem eine Öffnung 49 ausgebildet ist. Auf dem Substrat ist weiterhin eine erste Elektrode 42 angeordnet.

Unter dem Substrat 40 ist ein Glasröhrchen 45 befestigt, an das sich eine Elektrode 43 mit Halterung anschließt.

Weiterhin umfasst die Messsonde eine Einrichtung zum Erzeugen von Unterdruck in dem Glasröhrchen, die durch das Bezugszeichen 46 angedeutet ist.

Neben der dargestellten Ausführungsform der Messsonde, die nicht als Beschränkung der vorliegenden Erfindung zu verstehen ist, sind weitere Abwandlungen möglich.

Als Biochips lassen sich beispielsweise beliebige der erfindungsgemäßen Biochips einsetzen. Insbesondere die Abmessungen bestimmen sich hierbei nach dem Einsatzgebiet, also insbesondere der Zahl der zu untersuchenden Kanäle.

Das Glasröhrchen kann beispielsweise mit dem Substrat verklebt werden.

Die Elektrodeneinrichtung mitsamt Halterung kann so ausgebildet werden, dass sie von unten in das Glasröhrchen einschraubbar ist.

Weiterhin kann zwischen der Öffnung des Glasröhrchens und der einschraubbaren Elektrode ein Dichtungsring vorgesehen sein.

Im folgenden wird beschrieben, wie mit der vorliegenden Messsonde lonenströme durch den lonenkanal gemessen werden können.

Zunächst wird eine Zellmembran in Elektrolytlösung auf das Substrat aufgebracht. Durch Betätigung der Einrichtung zum Erzeugen von Unterdruck 46 wird die Membran mitsamt dem lonenkanal in die Öffnung gesaugt. In der Messsonde befindet sich ebenfalls eine Elektrolytlösung 44. Über die beiden Elektroden 42 und 43 kann schließlich der durch den lonenkanal fließende Strom gemessen werden.

## Patentansprüche

1. Biochip (1; 2; 3) zur Untersuchung von lonenkanälen, mit einem Substrat (10; 20; 30), in welchem Öffnungen (19; 29; 39) zur Aufnahme einer wenigstens einen lonenkanal (I) umfassenden Zellmembran (Me) oder einer künstlichen wenigstens einen lonenkanal umfassenden Lipidmembran in Form einer M×N Matrix vorgesehen sind, wobei M ≥ 1 und N ≥ 1.

2. Biochip (1; 2; 3) nach Anspruch 1, in welchem die Oberfläche des Biochips im Bereich einer jeden Öffnung aufnahmeseitig eine Einrichtung zur Verbesserung des Kontaktes von Zellmembran und Biochip aufweist.

3. Biochip nach Anspruch 2, in welchem die Einrichtung zur Verbesserung des Kontaktes in Form einer Strukturierung der Oberfläche ausgebildet ist.

4. Biochip nach Anspruch 3, in welchem die Strukturierung in Form eines oder einer Mehrzahl von Ringen, der oder die um jede Öffnung angeordnet sind, oder in Form eines oder einer Mehrzahl von Quadraten oder Rechtecken, das oder die um jede Öffnung angeordnet sind, vorgesehen ist.

5. Biochip nach einem der Ansprüche 1 bis 4, in welchem jede Öffnung im wesentlichen kreisförmig ist.

6. Biochip nach einem der vorangegangenen Ansprüche, in welchem das Substrat einen Basisabschnitt (10; 20; 30) mit einer ersten Dicke (d₁) und im Basisabschnitt ausgebildete Fensterabschnitte (11; 21; 31) mit einer zweiten Dicke (d₂) aufweist, wobei jede Öffnung in einem entsprechenden Fensterabschnitt vorgesehen ist.

7. Biochip nach einem der vorangegangenen Ansprüche, in welchem das Substrat ein Halbleitermaterial, wie GaAs, Si, oder AlGaAs oder Quarz oder Polymere, wie Polydimethylsiloxan (PDMS) umfasst.

8. Biochip nach einem der vorangegangenen Ansprüche, in welchem Elektroden auf einer oder auf beiden Seiten des Substrats vorgesehen sind.

9. Biochip nach einem der vorangegangenen Ansprüche, in welchem aktive und/oder passive Bauelemente auf dem Substrat integriert sind.

10. Biochip nach einem der vorangegangenen Ansprüche, in welchem die aktiven und/oder passiven Bauelemente eine Feldeffektverstärkereinrichtung zur Vorverstärkung von Messsignalen aufweist.

11. Biochip nach einem der vorangegangenen Ansprüche, in welchem eine optische Nahfeldeinrichtung zur Beobachtung des oder der lonenkanäle vorgesehen ist.

12. Biochip nach Anspruch 11, in welchem die optischen Nahfeldeinrichtungen Rastersondeneinrichtungen umfassen.

13. Biochip nach einem der vorangegangenen Ansprüche, in welchem Mikrofluidkanäle zur on-chip Perfusion vorgesehen sind.
